(19) Europäisches Patentamt
European Patent Office
Office européen des brevets

(11) Publication number : **0 495 638 A2**

(12) # EUROPEAN PATENT APPLICATION

(21) Application number : **92300330.5**

(22) Date of filing : **15.01.92**

(51) Int. Cl.$^5$ : **C12N 15/12,** C07K 13/00, C12N 15/70, C12N 1/21, // A61K37/02 , (C12N1/21, C12R1:19)

The microorganism(s) has (have) been deposited with American Type Culture Collection under number ATCC 55132.

(30) Priority : **16.01.91 US 641345**

(43) Date of publication of application : **22.07.92 Bulletin 92/30**

(84) Designated Contracting States : **AT BE CH DE DK ES FR GB GR IT LI LU MC NL PT SE**

(71) Applicant : **SCHERING CORPORATION**
**2000 Galloping Hill Road**
**Kenilworth New Jersey 07033 (US)**

(72) Inventor : **Alexander, Denise M.**
**3414 Shelton Avenue**
**Bethlehem, Pennsylvania 18017 (US)**
Inventor : **Cable, Michael B.**
**3 Forsgate Drive**
**Freehold, New Jersey 07728 (US)**
Inventor : **Dalie, Barbara L.**
**900 Briarcliff Avenue**
**Maywood, New Jersey 07607 (US)**
Inventor : **Narula, Satwant K.**
**26 Natalie Drive**
**West Caldwell, New Jersey 07006 (US)**

(74) Representative : **Ritter, Stephen David et al**
**Mathys & Squire 10 Fleet Street**
**London EC4Y 1AY (GB)**

(54) **Expression of mature PDGF-B in Escherichia coli.**

(57) Recombinant vectors which encode mature human PDGF-B and *E. coli* bacteria transformed with such vectors are provided by this invention. These bacteria lack the lon protease (*lon*$^-$) and the heat shock regulatory factor *htp*R (*htp*R$^-$). Also provided are methods for using such transformed bacteria to make unglycosylated, biologically active, mature human PDGF-B.

EP 0 495 638 A2

## BACKGROUND OF THE INVENTION

Platelet-derived growth factor (PDGF) is a major mitogen in serum that promotes the proliferation of fibroblasts and smooth muscle cells *in vitro* [Heldin *et al.*, Cell *37*:9 (1984); Deuel et *al.*, Curr. Top. Cell. Regul. 26:51 (1985); Ross *et al.*, Cell *46*:155 (1986)]. PDGF produces its mitogenic effects by binding with high affinity to specific cellular receptors which are stimulated to autophosphorylate tyrosine residues.

Purified PDGF is a cationic glycoprotein having a molecular weight of about 30,000 daltons, although the molecule exhibits considerable size heterogeneity. Sizes of individual species range from about 27,000 to 31,000 daltons. This heterogeneity is believed to result from differing degrees of glycosylation and from the presence of two forms of polypeptide chains, A and B. All of the PDGF species exhibit identical biological activities which are abolished by chemical cleavage of disulfide bridges.

Natural PDGF is normally a heterodimer composed of one A chain and one B chain which are linked by such bridges, although homodimers containing two A chains or two B chains are also known [Hart *et al.*, Biochemistry *29*:166 (1990)]. Kelly *et al.* [EMBO J. *4*:3399 (1985)], have expressed derivatives of the v-*sis* gene (which encodes $p^{28sis}$) in *S. cerevisiae* to produce polypeptides containing only the B chain sequences. The expression products were biologically active, showing that the B chain of PDGF alone is sufficient for mitogenesis. More recently, Hoppe *et al.* [Biochemistry *28*:2956 (1989)] have described the preparation of a modified recombinant PDGF-B polypeptide which formed a homodimer and displayed biological activity.

Amino acid sequence and cDNA sequence data show that the A and B chains of PDGF are partially homologous, and that the B chain is highly homologous to a portion of the predicted amino acid sequence of $p^{28sis}$, the transforming product of the simian sarcoma virus [Doolittle *et al.*, Science *221*:275 (1983); Waterfield *et al.*, Nature *304*:35 (1983)]. Wang *et al.* [J. Biol. Chem. *259*:10645 (1984)] have also shown that $p^{28sis}$ competes with $^{125}$I-PDGF for binding to PDGF receptor sites in fibroblast membranes.

Because of its mitogenic effects, PDGF can be used to promote wound healing. It would therefore be desirable to produce sufficient quantities of PDGF for medical use. Preferably, such PDGF should have a well-defined molecular structure and be a single species, instead of a mixture of species such as is found in natural sources.

In an effort to produce large quantities of a PDGF-B homolog, Giese *et al.* [J. Virol. *63*:3080 (1989)] expressed a monkey v-*sis* gene using a baculovirus vector system. The result, however, was a heterogeneous mixture due apparently to variable glycosylation and to proteolytic processing at both the amino and carboxyl termini.

Glycosylation of PDGF is not required for biological activity, and production of the unglycosylated protein would be desirable because it would avoid the microheterogeneity caused by variations in glycosylation. Attempts to produce unglycosylated, biologically active, mature human PDGF in bacteria such as *E. coli*, however, have been unsuccessful. Early efforts produced material that was biologically inactive, presumably due to incorrect polypeptide chain folding and disulfide bridge formation in the microbial environment [Devare *et al.*, Cell *36*:43 (1984); Wang *et al.*, J. Biol. Chem. *259*:10645 (1984)]. More recent efforts have produced biologically active material in an *E. coli* expression system, but not intact, mature protein containing all of the PDGF sequence and no extraneous sequences.

For example, Hoppe *et al., supra*, prepared modified biologically active PDGF-B dimers by expressing a gene encoding a cro-β-gal-PDGF-B fusion protein in *E. coli.* The fusion protein produced, upon cleavage with cyanogen bromide, resulted in a truncated form of PDGF-B in which twelve amino acid residues were missing from the amino terminus. The PDGF-B of Hoppe *et al.* also contained a five-amino acid extension of the carboxyl terminus, the last three residues of which were unrelated to any PDGF sequences and originated from a stop linker fused to the PDGF cDNA.

## SUMMARY OF THE INVENTION

The present invention provides unglycosylated, biologically active, mature human PDGF-B. In a preferred embodiment this PDGF-B is substantially free of bacterial proteins.

This invention further provides DNA and recombinant vectors encoding such PDGF-B. These vectors comprise DNA encoding mature human PDGF-B and are capable of directing expression of such DNA in an *E. coli* bacterium. Preferably, the vectors comprise, from 5′ to 3′, a *Tac* promoter, an $I^q$ repressor gene and DNA encoding mature, human PDGF-B.

This invention still further provides *E. coli* bacteria transformed with recombinant vectors comprising DNA encoding mature human PDGF-B, which bacteria are capable of expressing such DNA. These bacteria lack the *lon* protease (*lon*$^-$) and the heat shock regulatory factor *htp*R (*htp*R$^-$).

This invention still further provides a method for making biologically active, unglycosylated, mature human PDGF-B comprising:

(a) culturing a *lon*⁻, *htp*R⁻ *E. coli* bacterium transformed with a recombinant vector comprising DNA encoding mature human PDGF-B, under conditions in which the DNA is expressed; and

(b) preparing biologically active, unglycosylated, mature human PDGF-B from the culture.

## BRIEF DESCRIPTION OF THE FIGURES

This invention can be more readily understood by reference to the accompanying figures, in which:

Fig. 1 is a schematic representation of plasmid pTacBI$^q$.

Fig. 2 is a graphical representation of the results of a mitogenesis assay in which the unglycosylated PDGF-B of the invention and various commercial PDGFs were examined for their ability to stimulate DNA synthesis in human foreskin fibroblasts. Incorporation of $^3$H-thymidine into DNA is shown as a function of the concentration of the PDGF-B of the invention (open squares) and PDGF-Bs from Bachem Bioscience Inc. (open circles), Collaborative Research, Inc. (closed circles) and Genzyme Corp. (open squares).

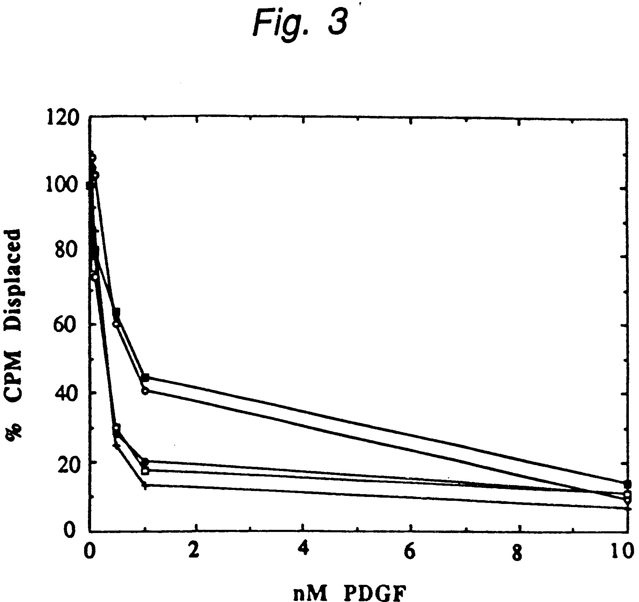

Fig. 3 is a graphical representation of the results of a radioligand-receptor assay in which the unglycosylated PDGF-B of the invention and various commercial PDGFs were examined for their ability to inhibit the specific binding of $^{125}$I-PDGF-B to receptors on human foreskin fibroblasts. The percentage of inhibition of the labeled PDGF binding is shown as a function of the concentration of the PDGF-B of the invention (open squares) and PDGF-Bs from Upstate Biotechnology, Inc. (+), Bachem Bioscience Inc. (open circles), Collaborative Research, Inc. (closed circles) and Genzyme Corp. (open squares).

## DESCRIPTION OF THE INVENTION

All references cited herein are hereby incorporated in their entirety by reference. All nucleic acid sequences disclosed follow the normal 5′ to 3′ convention, as read from left to right. Standard single-letter abbreviations are used for the nucleotide bases in the sequences (37 C.F.R. § 1.822).

As used herein, the term mature human PDGF-B" is defined as PDGF-B which (a) has an amino acid sequence substantially identical to the sequence defined by SEQ ID NO:1 and (b) has biological activity that is common to native PDGF-B. Substantial identity of amino acid sequences means that the sequence of another mature human PDGF-B protein compared to the sequence defined by SEQ ID NO:1 is identical or differs by one or more amino acid alterations (deletions, additions, substitutions) that do not substantially impair biological activity.

For example, there may be allelic variants of the sequence defined by SEQ ID NO:1. Furthermore, it is well within the skill of the art, *e.g.*, by chemical synthesis or by the use of modified polymerase chain reaction (PCR) primers or site-directed mutagenesis to modify DNA encoding PDGF-B having the sequence defined by SEQ ID NO:1 to produce single or multiple base substitutions which do not substantially impair the biological activity of PDGF-B produced therefrom. Such conservatively modified variants are within the scope of this invention.

The amino acid sequence defined by SEQ ID NO:1 is identical to a subsequence of the c-*sis* protein disclosed by Ratner *et al.* [Nucleic Acids Res. *13*:5007 (1985)].

Although for convenience the biologically active PDGF of this invention is referred to herein as "PDGF-B", as noted above this protein is actually a homodimer comprising two B chains (*i.e.*, PDGF-BB).

DNA encoding the PDGF-B of this invention can be prepared by chemical synthesis using the known nucleic acid sequence [Ratner *et al.*, Nucleic Acids Res. *13*:5007 (1985)] and standard methods such as the phosphoramidite solid support method of Matteucci *et al.* [J Am. Chem. Soc. *103*:3185 (1981)] or the method of Yoo *et al.* [J. Biol. Chem. *764*: 17078 ( 1989)].

Alternatively, cDNA can be made from any cell known to produce PDGF. Such cells include, *e.g.*, human sarcoma, glioblastoma, placental and venous endothelial cells. Human T-lymphoid cell lines produced by transformation by HTLV-I or -II (*e.g.*, HUT 102 cells) are another source. Messenger RNA (mRNA) can be isolated from these cells using standard techniques, and this mRNA can be used as a template to produce double-stranded cDNA as described by Maniatis *et al.*, Molecular Cloning: A Laboratory Manual, 1982, Cold Spring Harbor Laboratory, Cold Spring Harbor, NY. This cDNA can then be inserted into a cloning vector which can be used to transform *E. coli* to produce a cDNA library.

The cDNA library can then be screened using molecular probes based on the known nucleic acid sequence Such probes can be radiolabeled, *e.g.*, by nick-translation using *Pol*I DNA polymerase in the presence of the four deoxynucleotides, one of which contains $^{32}$P in the $\alpha$ position (Maniatis *et al., supra*, p. 109).

Yet another method was used to obtain such DNA in the Example below. There, the PCR method [Saiki *et al.*, Science *239*:487 (1988)] was used, with oligonucleotide primers based upon the known PDGF-B sequence and a plasmid containing a PDGF-B cDNA insert as a template, to make copies of PDGF-B DNA. The PCR method can also be applied to cDNA libraries and to other plasmids known in the art to contain such DNA.

Of course, because of the degeneracy of the genetic code, there are many functionally equivalent nucleic acid sequences that can encode mature human PDGF-B as defined herein. Such functionally equivalent sequences, which can readily be prepared using known methods such as chemical synthesis, PCR employing modified primers and site-directed mutagenesis, are within the scope of this invention.

Insertion of DNA encoding human PDGF-B into a vector is easily accomplished when the termini of both the DNA and the vector comprise the same restriction site. If this is not the case, it may be necessary to modify the termini of the DNA and/or vector by digesting back single-stranded DNA overhangs generated by restriction endonuclease cleavage to produce blunt ends, or to achieve the same result by filling in the single-stranded termini with an appropriate DNA polymerase. Alternatively, any site desired may be produced by ligating nucleotide sequences (linkers) onto the termini. Such linkers may comprise specific oligonucleotide sequences that define desired restriction sites. The cleaved vector and the DNA fragments may also be modified if required by homopolymeric tailing.

Many *E. coli*-compatible expression vectors can be used in this invention, including but not limited to vectors containing bacterial or bacteriophage promoters such as the *Tac*, *Lac*, *Trp*, *Lac*UV5, $\lambda$ $P_r$ and $\lambda$ $P_L$ promoters. Preferably, a vector selected will have expression control sequences that permit regulation of the rate of PDGF-B DNA expression. Then, PDGF-B production can be regulated to avoid overproduction that could prove toxic to the host cells. Most preferred is a vector comprising, from 5′ to 3′ (upstream to downstream), a *Tac* promoter, an $I^q$ repressor gene and DNA encoding mature human PDGF-B. The vectors chosen for use in this invention may also encode secretory leaders such as the ompA or protein A leader, as long as such leaders are cleaved during post-translational processing to produce mature PDGF-B.

In the Example below, the *Tac* promoter, $I^q$ repressor gene and PDGF-B DNA were obtained using plasmids pTacRBS, piql-1 and pSM-1, respectively, to make final exemplary plasmid pTacB$I^q$. Those skilled in the art will know, however, that there are other readily available sources of the elements needed to make pTacB$I^q$.

For example, in an alternative route to plasmid pTacB$I^q$, plasmid pBTac2 (source of the *Tac* promoter; available, *e.g.*, from Boehringer Mannheim) is prepared by *Bam* HI digestion, blunt-ending with Mung bean nuclease, cleavage with *Pst* I and dephosphorylation with calf intestinal alkaline phosphatase. The large fragment is isolated and purified from an agarose gel.

To generate a DNA fragment encoding the mature form of human PDGF-B, two oligodeoxynucleotides having nucleic acid sequences defined by SEQ ID NO:2 and SEQ ID NO:3 are synthesized and used as primers for PCR with $\lambda$ phage DNA (isolated from a human placental cDNA library) as template. The resulting human PDGF-B DNA fragment is digested with *Pst* I and ligated to prepared plasmid pBTac2. The resulting plasmid containing the PDGF-B sequence in the correct orientation is designated pTacB.

An 1107-nucleotide *Eco* RI fragment encompassing the *E. coli Lac* $I^q$ gene is generated from *E. coli* K-12 strain JM101 (ATCC 33876) DNA by PCR using synthetic oligodeoxyn ucleotides having nucleic acid sequences defined by SEQ ID NO:4 and SEQ ID NO:5 as primers. After *Cla* I digestion, the *Lac* $I^q$ fragment is ligated to *Cla* I-digested, dephosphorylated pTacB DNA. Following transformation of *E. coli*, positive clones are identified and the orientation of the *Lac* $I^q$ gene is determined by endonuclease digestion.

Alternatively, a wild-type *Lac* promoter can be isolated from any strain and then mutated using, *e.g.*, PCR with primers incorporating the single-base substitution known to be present in the $I^q$ gene [Muller-Hill *et at.*, Proc. Natl. Acad. Sci. USA *59*: 1259 ( 1968)].

Doubly-mutated torn, *htp*R$^-$ *E. coli* bacteria for use in this invention can be produced using readily available singly-mutated bacteria as described in detail below. Transformation of the recombinant vectors of the invention into such bacteria can be carried out using standard methods.

The transformed bacteria are then cultured under conditions in which the PDGF-B cDNA is expressed, and biologically active human PDGF-B is prepared from the culture by a process comprising:

(a) disrupting the cells;

(b) preparing an inclusion body fraction from the disrupted cells;

(c) solubilizing the PDGF-B in the inclusion body fraction of step (b) using a concentrated urea solution; and

(d) refolding the solubilized PDGF-B from step (c), using a mixture of reduced and oxidized glutathione and a gradient of decreasing urea concentration.

As produced in *E. coli*, the PDGF-B remains in the cytoplasm in inclusion (refractile) bodies. To free the PDGF it is thus necessary to disrupt the membranes of the cells. This is preferably accomplished by sonication or by other mechanically disruptive means, such as a French pressure cell or Gaulin homogenizer.

Cell disruption can also be accomplished by chemical or enzymatic means. Since divalent cations are often required for cell membrane integrity, treatment with appropriate chelating agents such as EDTA or EGTA may prove sufficiently disruptive to the membranes. Similarly, enzymes such as lysozyme have been used to achieve the same result. That enzyme hydrolyzes the peptidoglycan backbone of the cell wall.

The application of osmotic shock can also be employed. This is accomplished by first placing the cells in a hypertonic solution which causes them to lose water and shrink. Subsequent placement in a hypotonic "shock" solution causes a rapid influx of water into the cells and a bursting of the cells.

Once the cells are disrupted, cellular debris is removed and an inclusion body fraction is obtained by standard methods. PDGF-B in this fraction is solubilized, using a urea solution of about 7 or 8 M, preferably 8 M, concentration. A concentrated (about 6 M) guanidine-HCl solution could be used instead, but urea is preferred because it permits the use of cation exchange chromatography for subsequent purification of the solubilized protein.

The solubilized protein is purified by standard methods such as by adsorption chromatography, cation or anion exchange chromatography, gel filtration chromatography, preparative electrophoresis in polyacrylamide gel or other supporting matrices, isoelectric focusing etc. Cation exchange chromatog raphy using salt-gradient elution is preferred, with carboxymethyl-Sepharose® (CM-Sepharose®; a beaded agarose product of Pharmacia Fine Chemicals) the resin of choice. Of course other CM-substituted resins such as CM-cellulose can be used as well.

Following purification, the PDGF-B is sulfonated by standard methods, preferably using about 0.1 M sodium sulfite and 1 mM sodium tetrathionite. Refolding of the sulfonated PDGF-B into a biologically active form is initiated in concentrated, preferably 8 M, urea containing a mixture of the reduced and oxidized forms of glutathione (GSH). The molar ratio of the reduced:oxidized GSH can vary from about 10:1 to 6:1, with the concentration of the reduced form ranging from about 2 mM to 10 mM. Preferably, a mixture of 5 mM reduced GSH and 0.5 mM oxidized GSH is used.

Refolding of the polypeptide chains into biologically active PDGF-B dimers is accomplished by maintaining the GSH concentrations while slowly decreasing the urea concentration to below 1 M, preferably to about 0.5 M. Although this can be accomplished in a number of ways, gradual withdrawal of the urea solution with a corresponding replacement by equal volumes of a urea-free solution is a convenient method. In the Example below, the reduction in urea concentration was achieved exponentially over a period of 70 hours. Preferably, the refolding procedure is carried out under an inert atmosphere such as argon gas.

The refolded PDGF-B can be freed of the urea and GSH by standard methods such as by ultrafiltration or dialysis against a desired buffer, or by the use of gel filtration or another chromatographic method.

The unglycosylated, mature human PDGF-B of this invention can be used for any medical condition susceptible to treatment by the PDGFs of the prior art. Pharmaceutical compositions for such uses comprise an effective amount of unglycosylated, mature human PDGF-B and a physiologically acceptable carrier.

For example, formulations and dosage ranges for wound healing preparations containing PDGF are well known in the art (see, *e.g.*, U.S. patent No. 4,845,075 to Murray *et al.*). Also well known are compositions containing PDGF for use in treating skin damaged by cuts, abrasions, sun, wind and the like (see U.S. patent No 4,900,541 to Govier).

## EXAMPLE

The present invention can be illustrated by the following, non-limiting example. Unless otherwise specified, percentages given below for solids in solid mixtures, liquids in liquids, and solids in liquids are on a wt/wt, vol/vol and wt/vol basis, respectively.

### Materials Used

Restriction enzymes and T4 DNA ligase were purchased from New England Biolabs, Beverly, MA; *Thermus aquaticus (Taq)* DNA polymerase was obtained from Beckman Inc., Fullerton, CA; and calf intestinal alkaline phosphatase (CIAP) and alkaline phosphatase-conjugated rabbit anti-goat IgG were supplied by Boehringer Mannheim Biochemicals, Indianapolis, IN. Goat anti-human PDGF polyclonal antiserum and PDGF standards were obtained from Collaborative Research, Inc., Bedford, MA. All enzymes were used in accordance with the manufacturers instructions. The Sequenase version 2.0 sequencing system was obtained from United States Biochemical, Cleveland, OH.

Plasmids pTacRBS [Zurawski *et al.*, J. Immunol. *137*:3354 (1986)], pSM-1 [Ratner *et al.*, Nucleic Acids Res. *13*:5007 (1985)] and pi$^{q}$I-1 were used as sources of the portable *Tac* promoter, c-*sis* cDNA and the *Lac* I$^{q}$ gene, respectively.

*E. coli* K-12 strains JM101 (ATCC 33876) and MM294 (ATCC 33625) were obtained from the American Type Culture Collection, Rockville, MD. Bacteriophage P1 (ATCC 25404-B1) is also available from this source. *E. coli* K-12 strains SG20252 (*lon*$^-$) and K165 (*hpt*R$^-$) were obtained from the *E. coli* Genetic Stock Center in the Department of Biology at Yale University, New Haven, CT. A *lon*$^-$, *htp*R$^-$ *E. coli* K-12 mutant designated CAG629 was a gift from Dr. C. Gross, University of Wisconsin, Madison, Wisconsin.

Oligonucleotide Synthesis

Oligonucleotides used for PCR were synthesized by standard methods using an Applied Biosystems 380B DNA synthesizer and purified by standard methods.

Construction of Plasmid pTacBI$^q$

Standard recombinant DNA methods were carried out essentially as described by Maniatis *et al.*, Molecular Cloning: A Laboratory Manual, 1982, Cold Spring Harbor Laboratory.

PCR reactions were performed with a Techne programmable Dri-Block (GRI, Essex, UK) essentially as described by Friedman *et al.* [Nucleic Acids Res. *16*:8718 (1988)]. Briefly, a reaction mixture consisting of 50 mM KCl, 10 mM Tris-HCl (pH 8.3), 1.5 mM $MgCl_2$, 1 mM dNTPs, 0.01% gelatin, 100 pmoles of each oligonucleotide primer, 50-100 ng of the appropriate template DNA , and 4-8 units of *Taq* polymerase, was subjected to thirty 2-minute cycles of 95, 50 and 72°C, with a final extension period of 9 minutes at 72°C. At the end of the incubation, the PCR mixture was subjected to electrophoresis in a 1.2% agarose/Tris-acetate gel. The PCR fragments of interest were visualized by staining with ethidium bromide and purified by gel electroelution. Following gel purification, the PCR fragments were digested with restriction endonucleases as indicated below.

To construct plasmid pTacBI$^q$, plasmid pTacRBS was prepared by digesting the plasmid with *Sac* I. The cleaved plasmid was then blunt-ended with T4 DNA polymerase, cleaved with *Bam* HI and dephosphorylated with CIAP, and the large fragment was isolated and purified by agarose gel electrophoresis.

To produce a DNA fragment encoding the mature form of human PDGF-B, plasmid pSM-1 was employed as a template and subjected to PCR using synthetic oligodeoxynucleotides having nucleic acid sequences defined by SEQ ID NO:6 and SEQ ID NO:7 as primers. The resulting human PDGF-B DNA fragment was isolated, digested with *Bam* HI and ligated to the prepared pTacRBS vector to produce a plasmid designated pTacB.

An 1107-base pair *Eco* RI fragment containing the *E. coli Lac* I$^q$ gene was isolated from pi$^q$I-1 and ligated to plasmid pTacB which had been digested with *Eco* RI and treated with CIAP. Following transformation of the ligation mixture into *E. coli* strain 294 as described below, positive clones were identified and the orientation of the *Lac*I$^q$ gene was determined by restriction endonuclease digestion. A large-scale preparation of the desired transformant was obtained by CsCl/ethidium bromide centrifugation (Maniatis *et al.*, *supra*, page 93), and the authenticity of the PDGF cDNA insert was established by sequencing using the sequenase version 2.0 system. The plasmid thus obtained was designated pTacBI$^q$ (Fig. 1).

Nucleic acid sequence analysis performed on plasmid pTacBI$^q$ produced a sequence for the mature PDGF-BcDNA insert that is defined by SEQ ID NO:8. This sequence is identical to the sequence of nucleotide residues 361-687 shown in Fig. 2 of the reference of Ratner *et al.* [Nucleic Acids Res. *13*:5007 (1985)].

Preparation of a lon$^-$, htpR$^-$ E. coli Bacterium

Although a *lon*$^-$, *htp*R$^-$ E. coli mutant designated CAG629 was used below in this Example, an equivalent mutant that can be used in the methods of this invention to produce similar results was also prepared. To make this mutant, a P1 transducing phage stock was made on *E. coli* K-12 strain SG20252 (*lon*$^-$) using standard methods [Miller, Experiments in Molecular Genetics, 1972, Cold Spring Harbor Laboratories, Cold Spring Harbor, NY, pp. 201 -205]. The P1 stock was adsorbed to *E. coli* strain K165 (*hpt*R$^-$) cells grown in LB (Luria-Bertani) medium containing 10 mM $CaCl_2$ at 30°C for 30 minutes. Adsorption was stopped by the addition of 0.1 M sodium citrate, pH 8.0, and the cells were harvested by centrifugation.

The cells were resuspended in the 20 mM sodium citrate and plated onto LB medium containing tetracycline (35 μg/ml). Tetracycline-resistant colonies which were mucoid (characteristic phenotype of *lon*$^-$ mutants) were isolated and replated.

The isolated transductant colonies were examined for increased sensitivity to ultraviolet (UV) irradiation (another characteristic of the *lon*$^-$ mutation) by exposing the colonies to UV irradiation for 0-30 seconds, using a lamp placed 30 cm from the culture dish. The cells were tested for viability after the various UV doses. One clone designated DA16 showed UV sensitivity that was increased compared to parental strain K165 but similar to that of strain SG20252.

Cell Transformation

*E. coli* K-12 CAG629 cells were transformed essentially as described by Maniatis *et al.* [*supra*, page 250], except that the cells were heat shocked at 30°C for 2 minutes and incubated at 25°C after plating.

Production of Recombinant Human PDGF

*E. coli* CAG629 cells were grown in LB medium containing 100 μg/ml ampicillin and 20 μg/ml tetracycline in 500-ml cultures at 27°C to a density of 0.6 O.D. at 600 nm. The cultures were then shifted to 37°C and grown to an O.D. of 0.8-1.0, at which time 2 mM isopropyl β-D-thiogalactopyranoside (IPTG) was added and incubation was continued at 37°C for 2 hours.

Purification of PDGF

Bacteria cultured as described above were harvested by centrifugation and resuspended in 25 ml of 20 mM Tris-HCl, pH 7.8, 0.5 mM ethylenediaminetetraacetic acid (EDTA), 10 mM $MgCl_2$ per 500 ml of culture. Cells were disrupted by passage through a Microfluidics fluidizer at 9-10,000 psi. DNAase and RNAase (Sigma Chemical Co., St. Louis, MO) were added to final concentrations of 20 μg/ml and 10 μg/ml, respectively, and digestion was carried out for 30 minutes at 25°C.

Cellular debris was removed by centrifugation at 1000 x g for 5 minutes at 4°C. Inclusion bodies were obtained after centrifugation at 27,000 x g for 15 minutes at 4°C. The inclusion body pellet was resuspended in 40 ml of 2 M urea with 2% Triton X-100 and then pelleted as before. The washed pellet was resuspended in 20 ml of 30% sucrose and centrifuged at 10,000 x g for 15 minutes at 4°C. The final pellet was frozen at -70°C under $N_2$ gas prior to the subsequent purification of the PDGF-B.

In the purification steps described below, column fractions were analyzed by sodium dodecylsulfate polyacrylamide gel electrophoresis [SDS-PAGE; Laemmli, Nature *227*:680 (1970)] and immunoblotting [Towbin *et al.*, Proc. Natl. Acad. Sci. USA *76*:4350 (1979)] using goat anti-human PDGF polyclonal antiserum. Approximate protein measurements were made using the colorimetric reagent of Bio-Rad Labs [Bradford, Anal. Biochem. *72*:248 (1976)] and bovine serum albumin as a standard. More accurate protein determinations were made by amino acid composition analysis using standard methods.

Inclusion body pellets were extracted at room temperature with 25 mM MOPS (3-[N-Morpholino]propanesulfonic acid), pH 7.2, 8 M urea and 5 mM DTT (dithiothreitol). After clarifying the mixture by centrifugation at 25,000 x g for 45 minutes at 21°C, the supernatant fluid containing a total of 1106 mg of protein was applied to a 2.6 x 19.5 cm CM-Sepharose® column (Pharmacia LKB Biotechnology, Piscataway, NJ) that had previously been equilibrated with the extraction buffer. After washing the column with the equilibration buffer to remove unbound materials, the bound proteins were eluted with a linear 0 to 0.4 M NaCl gradient (14 bed volumes). Fractions containing PDGF were pooled and dialyzed against 6 M guanidine-HCl.

Proteins in the pooled fractions (about 28 mg) were sulfonated with 0.1 M sodium sulfite and 10 mM sodium tetrathionite for 5 hours at room temperature and dialyzed against 8 M urea with 0.5 mM oxidized glutathione and 5 mM reduced glutathione in 50 mM Tris-HCl, 2 mM EDTA, pH 7.8, under argon. The urea concentration was then decreased exponentially over 70 hours by pumping out the buffer containing urea at the same rate as buffer lacking urea was pumped in under argon. The dialyzed sample was then adjusted to 50 mM sodium acetate, pH 5.0, and 0.25 M NaCl, and any precipitate present was pelleted by centrifugation and discarded.

The supernatant fluid (containing primarily refolded dimer and monomer) was concentrated about 3X by ultrafiltration through an Amicon YM-5 membrane and then subjected to reversed-phase high-performance liquid chromatography (HPLC) on a C4 Dynamax® column (300 Å; n-butyltrichlorosilane resin from Rainin Instr. Co., Ridgefield, NJ). The sample was applied to the column in 0.1% trifluoroacetic acid (TFA) and eluted with a 0-60% acetonitrile gradient in 0.1% TFA (3 bed volumes). The final yield of HPLC-purified PDGF-B was about 2 mg, or 0.2% of the total starting amount of protein.

PDGF Characterization

Amino Acid Sequencing

Amino acid composition analysis was carried out using standard methods on a sample of the purified, refolded PDGF-B and on a sample of yeast-expressed recombinant human PDGF-B purchased from Genzyme Corporation, Boston, MA. The samples were hydrolyzed at 150°C in 6 N HCl for 1 hour in a Waters Pico-Tag work station. The amino acids were analyzed on a Hewlett-Packard Amino Quant work station by pre-column derivatization using OPA-Fmoc chemistry, and the OPA derivatives were separated by reverse-phased HPLC.

These analyses showed that the amino acid compositions of the two proteins were virtually identical. The analysis of the purified, refolded PDGF-B also showed that the actual protein concentration of the sample was exactly twice the value determined with the bio-Rad colorimetric assay. All colorimetric values were corrected accordingly.

Thirty cycles of N-terminal amino acid sequence analysis were also carried out on the purified, refolded PDGF-B protein by standard automated Edman degradation. An Applied Biosystems Model 477A protein sequencer was used, and the PHT-amino acids were separated on an Applied Biosystems Model 120A PHT analyzer. The sequence data produced by this analysis were in agreement with the known N-terminal sequence of human PDGF (SEQ ID NO:1). The amino acid sequence analysis also showed that a small amount of the purified protein (less than 10%) contained an additional N-terminal methionine residue.

Electrophoretic Analysis

SDS-PAGE and capillary zone electrophoresis carried out on a Applied Biosystems Model 270A unit in 200 mM sodium phosphate, pH 2.5, showed that the purified, refolded PDGF-B was greater than 90% pure.

Mitogenesis Assay

To determine whether the unglycosylated PDGF-B could induce mitogenesis in human fibroblasts, a sample of the purified, refolded material and samples of commercially-purchased PDGF from Genzyme Corporation (boston, MA), Bachem Bioscience Inc. (Philadelphia, PA) and Collaborative Research, Inc. (Bedford, MA) were assayed in parallel for mitogenic activity, essentially as described by Witte et al. [Circulation Res. *42*:402 (1978)].

Hs27 newborn human foreskin cells (ATCC CRL 1634) were plated in 24-well tissue culture dishes (Corning) at a density of 3 x $10^4$ cells per well in 0.5-ml volumes of a complete medium containing Dulbecco's Modified Eagle's Medium (DMEM), 10% fetal calf serum (FCS), 2 mM glutamine and 1 % streptomycin/penicillin solution (100 units penicillin and 100 μg streptomycin/ml). After incubation overnight in a humidified 5% $CO_2$ incubator at 37°C, the medium in the wells was replaced with medium containing 1% FCS and incubated until the cells became confluent.

Serial dilutions of the purified material and the commercial PDGF were added to the wells, and incubation was carried out overnight as above. $^3$H-thymidine (3 μCi, specific activity 6.7 μCi/mM; Amersham) was added to each well, and the plates were incubated for 4 hours at 37°C. Following this incubation, the medium was removed by aspiration, and the plates were washed in turn twice with phosphate-buffered saline, twice with 5% trichloroacetic acid and once with deionized water. One-ml aliquots of 0.2 N NaOH were added to each well, and 1 ml of the contents of each well was transferred to a scintillation vial containing 10 ml of Scintiverse II® (Fisher Scientific) cocktail and counted in a liquid scintillation spectrometer.

The results are shown in Fig. 2, where it can be seen that similar concentrations of the various PDGFs produced essentially similar increases in thymidine incorporation in the cultured cells. The unglycosylated PDGF-B of this invention was at least as active as the commercial PDGFs.

Displacement of $^{125}$I-PDGF-B from Cultured Fibroblasts

To determine whether the PDGF-B of this invention could also compete with and thereby inhibit the specific binding of PDGF to its cellular receptors, radioligand receptor binding assays were carried out essentially as described by Bowen-Pope *et al.* [Methods in Enzymology *109*:69 (1985)] on PDGF-B prepared as described above and on glycosylated PDGFs purchased from Genzyme Corporation, Upstate Biotechnology, Inc. (Lake Placid, NY), Bachem Bioscience Inc. and Collaborative Research, Inc.

Hs27 fibroblasts were plated on 24-well tissue culture dishes at a density of 3 x 104 cells/well in a complete medium containing DMEM, 10% FCS, 2 mM glutamine and 1% streptomycin/penicillin solution. After incubation overnight in a humidified 5% $CO_2$ incubator at 37°C, the medium in the wells was replaced with medium containing 0.5% FCS. The cells were incubated for at least 48 hours prior to use for assay.

Before the cells showed signs of deterioration (4 days), they were washed 3X with DMEM containing 1% bovine serum albumin (BSA) at 4°C. Serial dilutions of the various PDGFs together with 62 nCi of $^{125}$I-PDGF-B (Amersham; specific radioactivity about 800 Ci/mM) were then added to the wells in 0.2-ml volumes of the wash medium. After incubation at 4°C for 2 hours with agitation, the cells were washed 5X with DMEM containing 1% BSA, solubilized with 1 ml of 1% Triton X-100 and counted in an LKB Model 1275 gamma counter.

The results are shown in Fig. 3, where it can be seen that the unglycosylated PDGF-B of the invention (open boxes) was as effective as any of the other PDGFs in inhibiting the specific binding of the labeled PDGF to the receptors on the Hs27 cells.

## Culture Deposit

*E. coli* clone DA16 was deposited with the American Type Culture Collection, Rockville, MD, on December 19, 1990 and assigned accession No. ATCC 55132. This deposit was made under the provisions of the Budapest Treaty on the International Recognition of the Deposit of Microorganisms for the Purposes of Patent Procedures.

Many modifications and variations of this invention can be made without departing from its spirit and scope, as will become apparent to those skilled in the art. The specific embodiments described herein are offered by way of example only, and the invention is to be limited only by the terms of the appended claims.

SEQUENCE LISTING

(1) GENERAL INFORMATION:

(i) APPLICANT: Alexander, Denise
Cable, Michael
Dalie, Barbara
Narula, Satwant

(ii) TITLE OF INVENTION: Expression of Mature Human PDGF-B in *E. coli*

(iii) NUMBER OF SEQUENCES: 8

(iv) CORRESPONDENCE ADDRESS:

(A) ADDRESSEE: Schering-Plough Corporation

(B) STREET: One Giralda Farms

(C) CITY: Madison

(D) STATE: New Jersey

(E) COUNTRY: USA

(F) ZIP: 07940

(v) COMPUTER READABLE FORM:

(A) MEDIUM TYPE: Floppy disk

(B) COMPUTER: Apple Macintosh

(C) OPERATING SYSTEM: Macintosh 6.0.5

(D) SOFTWARE: Microsoft Word 4.00B

(vi) CURRENT APPLICATION DATA:

(A) APPLICATION NUMBER: To be assigned

(B) FILING DATE: To be determined

(C) CLASSIFICATION:

(vii) PRIOR APPLICATION DATA: None

(viii) ATTORNEY/AGENT INFORMATION:

(A) NAME: Dulak, Norman C.

(B) REGISTRATION NUMBER: 31,608

(C) REFERENCE/DOCKET NUMBER: JB0169

(ix) TELECOMMUNICATION INFORMATION:

(A) TELEPHONE: 201-822-7375

(B) TELEFAX: 201-822-7039

(C) TELEX: 219165

(2) INFORMATION FOR SEQ ID NO: 1:

(i) SEQUENCE CHARACTERISTICS:

(A) LENGTH: 109 amino acids

(B) TYPE: amino acid

(D) TOPOLOGY: linear

(ii) MOLECULE TYPE: peptide

(xi) SEQUENCE DESCRIPTION: SEQ ID NO: 1:

```
Ser Leu Gly Ser Leu Thr Ile Ala Glu Pro Ala Met Ile Ala Glu Cys
1               5                   10                  15
Lys Thr Arg Thr Glu Val Phe Glu Ile Ser Arg Arg Leu Ile Asp Arg
            20                  25                  30
Thr Asn Ala Asn Phe Leu Val Trp Pro Pro Cys Val Glu Val Gln Arg
        35                  40                  45
Cys Ser Gly Cys Cys Asn Asn Arg Asn Val Gln Cys Arg Pro Thr Gln
    50                  55                  60
Val Gln Leu Arg Pro Val Gln Val Arg Lys Ile Glu Ile Val Arg Lys
65                  70                  75                  80
Lys Pro Ile Phe Lys Lys Ala Thr Val Thr Leu Glu Asp His Leu Ala
                85                  90                  95
Cys Lys Cys Glu Thr Val Ala Ala Ala Arg Pro Val Thr
            100                 105
```

(2) INFORMATION FOR SEQ ID NO: 2:

(i) SEQUENCE CHARACTERISTICS:

(A) LENGTH: 15 base pairs

(B) TYPE: nucleic acid

(C) STRANDEDNESS: single

(D) TOPOLOGY: linear

(xi) SEQUENCE DESCRIPTION: SEQ ID NO: 2:

AGCCTGGGTT CCCTG 15

(2) INFORMATION FOR SEQ ID NO: 3:

(i) SEQUENCE CHARACTERISTICS:

(A) LENGTH: 33 base pairs

(B) TYPE: nucleic acid

(C) STRANDEDNESS: single

(D) TOPOLOGY: linear

(xi) SEQUENCE DESCRIPTION: SEQ ID NO: 3:

CTGCAGTCAG GTCACAGGCC GTGCAGCTGC CAC 33

(2) INFORMATION FOR SEQ ID NO: 4:

(i) SEQUENCE CHARACTERISTICS:

(A) LENGTH: 27 base pairs

(B) TYPE: nucleic acid

(C) STRANDEDNESS: single

(D) TOPOLOGY: linear

(xi) SEQUENCE DESCRIPTION: SEQ ID NO: 4:

ATCGATGACA CCATCGAATG GAAAACC 27

(2) INFORMATION FOR SEQ ID NO: 5:

(i) SEQUENCE CHARACTERISTICS:

(A) LENGTH: 33 base pairs

(B) TYPE: nucleic acid

(C) STRANDEDNESS: single

(D) TOPOLOGY: linear

(xi) SEQUENCE DESCRIPTION: SEQ ID NO: 5:

ATCGATTCAC TGCCCGCTTT CCAGTCGGGA AAC 33

(2) INFORMATION FOR SEQ ID NO: 6:

(i) SEQUENCE CHARACTERISTICS:

(A) LENGTH: 21 base pairs

(B) TYPE: nucleic acid

(C) STRANDEDNESS: single

(D) TOPOLOGY: linear

(xi) SEQUENCE DESCRIPTION: SEQ ID NO: 6:

AGCCTGGGTT CCCTGACCAT T 21

(2) INFORMATION FOR SEQ ID NO: 7:

(i) SEQUENCE CHARACTERISTICS:

(A) LENGTH: 42 base pairs

(B) TYPE: nucleic acid

(C) STRANDEDNESS: single

(D) TOPOLOGY: linear

(xi) SEQUENCE DESCRIPTION: SEQ ID NO: 7:

```
CCCGGAGGAT CCTCAGGTCA CAGGCCGTGC AGCTGCCACT GT   42
```

(2) INFORMATION FOR SEQ ID NO: 8:

(i) SEQUENCE CHARACTERISTICS:

(A) LENGTH: 327 base pairs

(B) TYPE: nucleic acid

(C) STRANDEDNESS: double

(D) TOPOLOGY: linear

(xi) SEQUENCE DESCRIPTION: SEQ ID NO: 8:

```
AGCCTGGGTT CCCTGACCAT TGCTGAGCCG GCCATGATCG CCGAGTGCAA GACGCGCACC  60
GAGGTGTTCG AGATCTCCCG GCGCCTCATA GACCGCACCA ACGCCAACTT CCTGGTGTGG 120
CCGCCCTGTG TGGAGGTGCA GCGCTGCTCC GGCTGCTGCA ACAACCGCAA CGTGCAGTGC 180
CGCCCCACCC AGGTGCAGCT GCGACCTGTC CAGGTGAGAA AGATCGAGAT TGTGCGGAAG 240
AAGCCAATCT TTAAGAAGGC CACGGTGACG CTGGAAGACC ACCTGGCATG CAAGTGTGAG 300
ACAGTGGCAG CTGCACGGCC TGTGACC                                     327
```

## Claims

1. Unglycosylated, biologically active, mature human PDGF-B.

2. A recombinant vector comprising, from 5′ to 3′, a *Tac* promoter, an $I^q$ repressor gene and DNA encoding mature, human PDGF-B, which recombinant vector is capable of directing expression of such DNA in an *E. coli* bacterium.

3. An *E. coli* bacterium transformed with a recombinant vector comprising, from 5′to 3′, a *Tac* promoter, an $I^q$ repressor gene and DNA encoding mature, human PDGF-B, which bacterium is capable of expressing such DNA.

4. The bacterium of claim 3 which lacks the *lon* protease and the heat shock regulatory factor *htp*R.

5. A method for making unglycosylated, biologically active, mature human PDGF-B comprising:
(a) culturing a *lon*$^-$, *htp*R$^-$ *E. coli* bacterium transformed with a recombinant vector comprising DNA encoding mature human PDGF-B, under conditions in which such DNA is expressed; and
(b) preparing unglycosylated, biologically active, mature human PDGF-B from the culture.

6. The method of claim 5 in which such PDGF-B is prepared by a process comprising:
(i) disrupting the transformed bacterium;
(ii) preparing an inclusion body fraction from the disrupted cells of step (i);
(iii) solubilizing PDGF-B in the inclusion body fraction of step (ii) with about 8 M urea;
(iv) sulfonating the solubilized PDGF-B; and
(v) refolding the solubilized, sulfonated PDGF-B in a mixture containing about 8 M urea initially and reduced and oxidized GSH in a molar ratio of reduced:oxidized GSH of from about 10:1 to 6:1, whereby the initial urea concentration is gradually reduced to less than 1 M.

7. *E. coli* DA16, deposited with the American Type Culture Collection and assigned accession number ATCC 55132.

8. A pharmaceutical composition comprising unglycosylated, biologically active, mature human PDGF-B and a physiologically acceptable carrier.

9. A process for preparing a pharmaceutical composition as claimed in claim 8 which comprises admixing unglycosylated, biologically active, mature human PDGF-B with a physiologically acceptable carrier.

10. The use of unglycosylated, biologically active, mature human PDGF-B for the preparation of a pharmaceutical composition for treating a medical condition susceptible to treatment by PDGF.

11. The use of unglycosylated, biologically active, mature human PDGF-B for treating a medical condition susceptible to treatment by PDGF.

*Fig. 1*

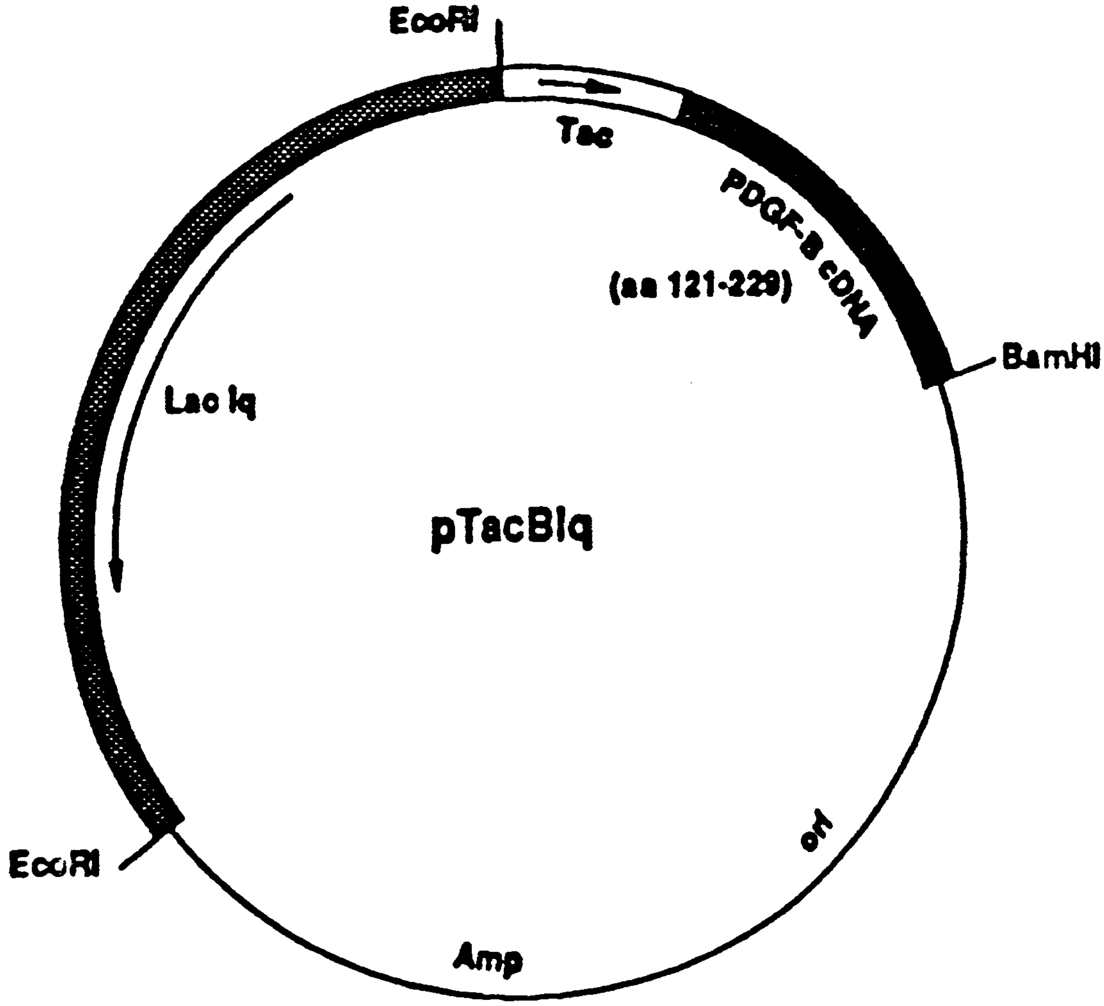
EcoRI
Tac
PDGF-B cDNA
(aa 121-229)
BamHI
Lac Iq
pTacBIq
EcoRI
ori
Amp

Fig. 2

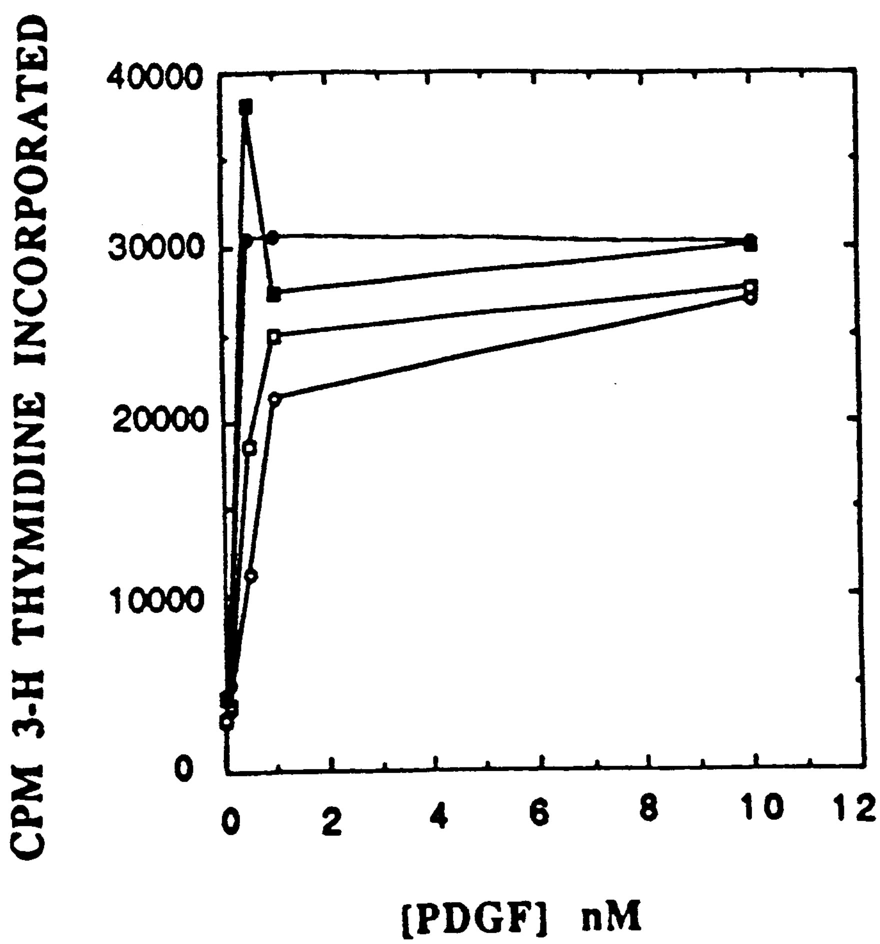
CPM 3-H THYMIDINE INCORPORATED
40000
30000
20000
10000
0
0
2
4
6
8
10
12
[PDGF] nM

Fig. 3